# EUROPEAN PATENT APPLICATION

(11) **EP 4 800 483 A1**
(43) Date of publication of application: **02.09.2026**
(21) Application number: 26159386.7
(22) Date of filing: 18.02.2026
(51) Int. Cl.: G03F 7/20

(54) **EXHAUST SYSTEM FOR EXHAUSTING HYDROGEN DISCHARGED FROM AN EUV EXPOSURE APPARATUS**

(30) Priority: 28.02.2025 JP 2025031336
(71) Applicant: EBARA CORPORATION, Ota-ku, Tokyo 144-8510 (JP)
(72) Inventor: SEGAWA, Yu, Tokyo, 1448510 (JP)
(74) Representative: Wagner & Geyer

(57) **Abstract**

An exhaust system for exhausting hydrogen discharged from an EUV exposure apparatus is provided that includes a light-source-unit hydrogen pump module having a vacuum pump unit for sucking hydrogen discharged from a light source unit of the EUV exposure apparatus, a first oxygen sensor module that measures an oxygen concentration of gas discharged from the light-source-unit hydrogen pump module, an exposure-unit hydrogen pump module having a vacuum pump unit for sucking hydrogen discharged from an exposure unit of the EUV exposure apparatus, a second oxygen sensor module that measures an oxygen concentration of gas discharged from the exposure-unit hydrogen pump module, a hydrogen treatment module for treating hydrogen gas discharged from the first and second oxygen sensor modules, a utility module for supplying cooling water and/or nitrogen gas to each module, and a control module for supplying power to each module, and controlling each module.

## Description

### BACKGROUND

### Technical Field

The present disclosure relates to an exhaust system for exhausting hydrogen and nitrogen discharged from an EUV exposure apparatus.

### Related Art

In recent years, an extreme ultra violet (EUV) exposure apparatus has been put into practical use that executes an exposure process on a substrate, such as a wafer, at an extremely short wavelength. The EUV exposure apparatus is an ultra-precision apparatus, and its performance will rapidly degrade due to, in particular, the entry of foreign matter into its optics. The EUV exposure apparatus includes a light source unit that generates EUV, and an exposure unit that exposes a substrate to the EUV generated by the light source unit. For the light source unit, oxide of tin (Sn) generated by the irradiation of a target with a laser beam is known as a representative contamination source, and for the exposure unit, an organic material desorbed from a photosensitive material (i.e., resist) is known as a representative contamination source.

As a countermeasure against such contamination, there is a method of using hydrogen gas. In the light source unit, hydrogen gas is used at a rate of several hundred L/min to remove oxide of tin as a gaseous hydride, and in the exposure unit, hydrogen gas is also used at a rate of several ten L/min to gasify an organic material for removal. The hydrogen gas used is mostly unreacted, but is discharged from the apparatus as a carrier of the removed contaminants. Therefore, exhaust gas containing a large amount of hydrogen gas, such as several hundred L/min, is discharged from the EUV exposure apparatus.

As an apparatus for treating hydrogen gas discharged from an EUV exposure apparatus, an exhaust system is known that exhausts hydrogen gas discharged from an EUV exposure apparatus, using a dry vacuum pump, and safely treats the exhausted hydrogen, using a hydrogen treatment apparatus (see JP 2022-42602 A).

### SUMMARY

[1] An exhaust system according to an aspect is an exhaust system for exhausting hydrogen and nitrogen discharged from an EUV exposure apparatus, the exhaust system including:
   a light-source-unit hydrogen pump module having a vacuum pump unit, the vacuum pump unit being configured to suck hydrogen discharged from a light source unit of the EUV exposure apparatus;
   a first oxygen sensor module configured to measure an oxygen concentration of gas discharged from the light-source-unit hydrogen pump module;
   an exposure-unit hydrogen pump module having a vacuum pump unit, the vacuum pump unit being configured to suck hydrogen discharged from an exposure unit of the EUV exposure apparatus;
   a second oxygen sensor module configured to measure an oxygen concentration of gas discharged from the exposure-unit hydrogen pump module;
   a hydrogen treatment module configured to treat hydrogen gas discharged from the first and second oxygen sensor modules;
   a utility module configured to supply cooling water and/or nitrogen gas to each module; and
   a control module configured to supply power to each module, and control each module.
[2] An exhaust system according to an aspect is the exhaust system according to [1] above, in which
   the hydrogen treatment module includes a hydrogen dilution apparatus configured to dilute the hydrogen gas, and
   air discharged from a housing of each module is supplied to the hydrogen dilution apparatus as dilution air.
[3] An exhaust system according to an aspect is the exhaust system according to [1] or [2] above, in which the hydrogen treatment module includes a combustion-type exhaust gas treatment apparatus configured to perform combustion treatment on the hydrogen gas.
[4] An exhaust system according to an aspect is the exhaust system according to any one of [1] to [3] above, further including a hydrogen recovery module configured to recover the hydrogen gas for reuse in the EUV exposure apparatus, in which
   the hydrogen recovery module includes a hydrogen purification apparatus configured to purify the recovered hydrogen gas.
[5] An exhaust system according to an aspect is the exhaust system according to any one of [1] to [4] above, further including a fuel cell module configured to generate power using the hydrogen gas.
[6] An exhaust system according to an aspect is the exhaust system according to any one of [1] to [5] above, in which the vacuum pump unit has a main pump, a first booster pump, and a second booster pump, and has a caster at a bottom.
[7] An exhaust system according to an aspect is the exhaust system according to any one of [1] to [6] above, further including a non-hydrogen pump module configured to discharge non-hydrogen gas discharged from a device other than the light source unit and the exposure unit of the EUV exposure apparatus.

### BRIEF DESCRIPTION OF DRAWINGS

FIG. 1 is a diagram illustrating an example of a schematic configuration of an exhaust system according to a first embodiment;
FIG. 2 is a diagram illustrating an example of a schematic configuration of a pump module according to the present embodiment;
FIG. 3 is a diagram illustrating a method of disposing a pump unit according to the present embodiment;
FIG. 4 is a diagram illustrating an example of a schematic configuration of an exhaust system according to a second embodiment; and
FIG. 5 is a diagram illustrating an example of a schematic configuration of an exhaust system according to a third embodiment.

### DETAILED DESCRIPTION

Hereinafter, each embodiment will be described with reference to the drawings. Note that unnecessarily detailed descriptions may be omitted. For example, a detailed description of a well-known matter and a repeated description of substantially the same configuration may be omitted. This is to avoid unnecessary redundancy of the following description and to facilitate understanding for those skilled in the art.

### <First embodiment>

Hereinafter, the present embodiment will be described with reference to the drawings. An exhaust system 10 according to an embodiment described below is used for treating hydrogen that has been used in an extreme ultraviolet exposure apparatus (hereinafter referred to as an EUV exposure apparatus) 1. The EUV exposure apparatus 1 typically includes a light source unit 2 that generates extreme ultraviolet (EUV), and an exposure unit 3 that exposes a workpiece, such as a substrate, a wafer, or a panel, to the extreme ultraviolet (EUV) generated by the light source unit 2. As described above, a large amount of hydrogen gas is used in the light source unit 2 and the exposure unit 3. Such hydrogen gas is sent to and treated by the exhaust system 10 described below.

### (Configuration of exhaust system)

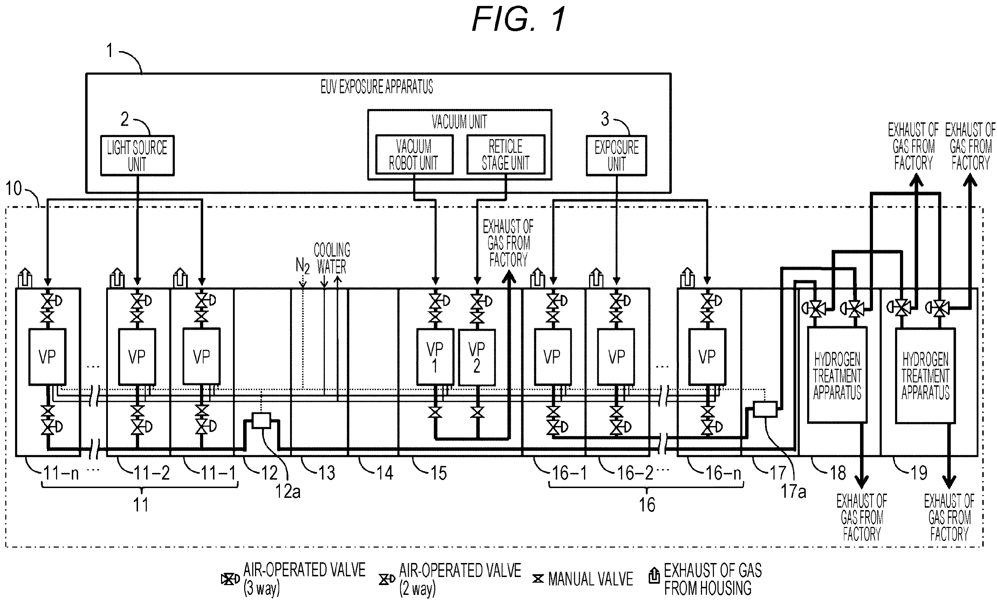

FIG. 1 is a diagram illustrating an example of a schematic configuration of the exhaust system 10. As illustrated in FIG. 1, the exhaust system 10 according to the present embodiment includes a plurality of modules. The number and types of the modules can be changed according to the needs of users. For example, the exhaust system 10 of the present embodiment includes a light-source-unit hydrogen pump module group 11 (which includes at least one hydrogen pump module and includes, for example, n hydrogen pump modules 11-1 to 11-n) that sucks hydrogen gas discharged from the light source unit 2 of the EUV exposure apparatus 1, a first oxygen sensor module 12 that measures the oxygen concentration of hydrogen gas discharged from the light-source-unit hydrogen pump module group 11, a utility module 13, a control module 14 that controls the entire exhaust system 10, a non-hydrogen pump module 15 for exhausting non-hydrogen gas (i.e., air and nitrogen), an exposure-unit hydrogen pump module group 16 (which includes at least one hydrogen pump module and includes, for example, n hydrogen pump modules 16-1 to 16-n) that sucks hydrogen gas discharged from the exposure unit 3 of the EUV exposure apparatus 1, a second oxygen sensor module 17 that measures the oxygen concentration of hydrogen gas discharged from the exposure-unit hydrogen pump module group 16, and a first hydrogen treatment module 18 and a second hydrogen treatment module 19 for treating hydrogen gas discharged from the light-source-unit hydrogen pump module group 11 and the exposure-unit hydrogen pump module group 16. Note that the light-source-unit hydrogen pump module group 11 and the exposure-unit hydrogen pump module group 16 basically have a similar configuration.

### (Housing exhaust 1: when each hydrogen treatment module includes hydrogen dilution apparatus)

Described first is the exhaust of gas from a housing when each of the first hydrogen treatment module 18 and the second hydrogen treatment module 19 includes a hydrogen dilution apparatus. Each of the light-source-unit hydrogen pump module group 11, the exposure-unit hydrogen pump module group 16, the first oxygen sensor module 12, the second oxygen sensor module 17, the utility module 13, the control module 14, and the non-hydrogen pump module 15 is provided with an exhaust port, which has a housing exhaust pipe (not illustrated) connected thereto as appropriate, and an air inlet port (not illustrated). Air exhausted from a housing of each of such modules can be used as dilution air for diluting hydrogen in a hydrogen dilution apparatus described later. Therefore, the user of the exhaust system 10 does not need to separately perform exhaust of gas from the housing for the purpose of diluting hydrogen.

In addition, suction pressure (i.e., air volume) is larger at housing exhaust portions closer to the hydrogen treatment modules 18 and 19, and suction pressure (i.e., air volume) is smaller at housing exhaust portions farther from the hydrogen treatment modules 18 and 19 due to the influence of conductance. Therefore, each exhaust port is provided with a damper to control a balance of the volume of air exhausted from each housing.

In addition, if particles, iron powder, and the like, which are charged, enter the hydrogen treatment modules 18 and 19, static electricity may be generated in the hydrogen treatment modules 18 and 19, may become an ignition source. Therefore, the exhaust port of each housing is provided with a filter to prevent such particles from entering the hydrogen treatment modules 18 and 19.

When air exhausted from the housing of each module is exhausted only via the hydrogen dilution apparatus, the exhaust of air from the housing cannot be continued if the hydrogen dilution apparatus (i.e., both the main apparatus and the spare apparatus) has stopped due to a failure during the discharge of hydrogen from the exposure apparatus. Under such circumstances, when there is a hydrogen leak in any of the modules, hydrogen will stay in the module. To prevent this, the housing exhaust pipe may be connected to at least one factory exhaust pipe. Accordingly, even in the above-described circumstances, it is possible to safely exhaust hydrogen from the housing.

### (Housing exhaust 2: when each hydrogen treatment module includes no hydrogen dilution apparatus)

Described next is the exhaust of gas from a housing when each of the first hydrogen treatment module 18 and the second hydrogen treatment module 19 includes no hydrogen dilution apparatus. Each of the light-source-unit hydrogen pump module group 11, the exposure-unit hydrogen pump module group 16, the first oxygen sensor module 12, the second oxygen sensor module 17, the utility module 13, the control module 14, and the non-hydrogen pump module 15 is provided with an exhaust port, which has a housing exhaust pipe (not illustrated) connected thereto as appropriate, and an air inlet port (not illustrated). Air in each module is discharged through the housing exhaust pipe. The housing exhaust pipe is connected to an exhaust facility (not illustrated) of a factory, and negative pressure is formed in the housing and in the housing exhaust pipe. Therefore, even if hydrogen leaks in the housing, leakage of hydrogen to the outside of the exhaust system is prevented. In addition, even if the housing exhaust pipe is damaged, leakage of hydrogen gas from the housing exhaust pipe is prevented.

### (Light-source-unit hydrogen pump modules)

Hydrogen gas discharged from the light source unit 2 of the EUV exposure apparatus 1 is sucked into the light-source-unit hydrogen pump module (i.e., a first hydrogen pump module) group 11 through a manifold. The light-source-unit hydrogen pump module group 11 includes a vacuum pump unit VP that sucks hydrogen gas discharged from the light source unit of the EUV exposure apparatus 1. A vacuum pump mounted in the vacuum pump unit VP is a positive-displacement dry vacuum pump, for example, but its specific configuration is not limited to a particular configuration. The exhaust system 10 of the present embodiment is configured such that hydrogen gas discharged from the light source unit 2 of the EUV exposure apparatus 1 is sucked into the light-source-unit hydrogen pump module group 11. The light-source-unit hydrogen pump module group 11 may include a plurality of hydrogen pump modules (it includes n hydrogen pump modules 11-1 to 11-n in the example illustrated in FIG. 1). Accordingly, even when the operation of one hydrogen pump module is stopped for maintenance, for example, the suction of hydrogen gas can be continued with the other hydrogen pump modules. Therefore, maintenance and the like of the hydrogen pump modules can be performed without stopping the operation of the EUV exposure apparatus 1.

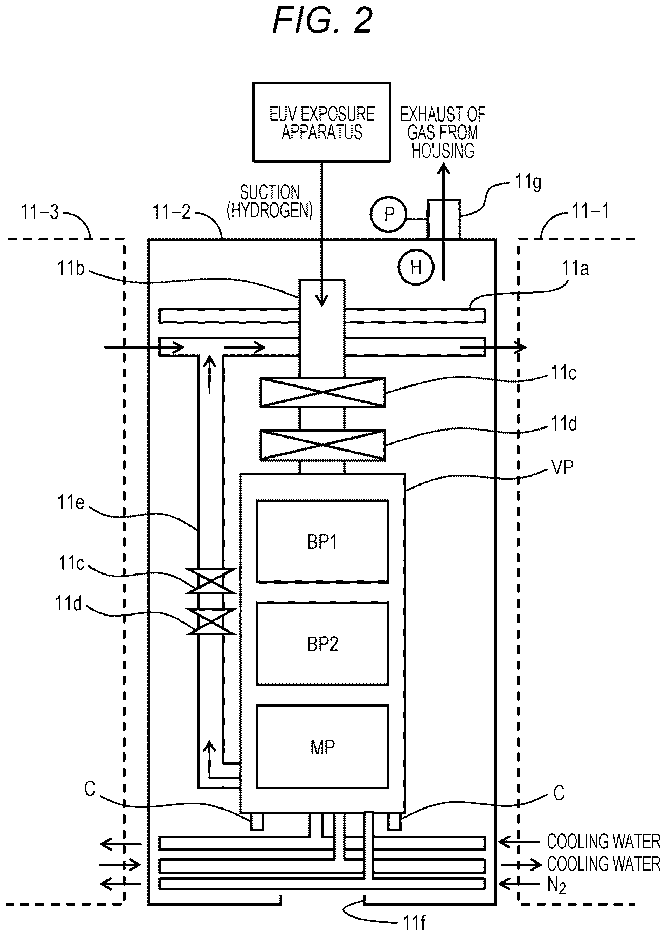

FIG. 2 is a diagram illustrating an example of a schematic configuration of a hydrogen pump module included in the light-source-unit hydrogen pump module group 11 of the present embodiment. As an example, the hydrogen pump module 11-2 included in the light-source-unit hydrogen pump module group 11 will be described, but the basic configuration is similar for the other hydrogen pump modules included in the light-source-unit hydrogen pump module group 11 and the hydrogen pump modules 16-1 to 16-n included in the exposure-unit hydrogen pump module group 16.

As illustrated in FIG. 2, the hydrogen pump module 11-2 stores a vacuum pump unit VP provided with a caster C. The vacuum pump unit VP is supplied with power from a power supply bus bar 11a.

The vacuum pump unit VP includes three vacuum pumps (i.e., a main pump MP, a first booster pump BP1, and a second booster pump BP2). As the three vacuum pumps of the vacuum pump unit VP are operated, hydrogen gas is sucked into the vacuum pump unit VP from the light source unit 2 of the EUV exposure apparatus 1 through an intake pipe 11b connected to an intake port. The intake pipe 11b is provided with an air-driven valve 11c and a manual valve 11d. At the time of replacement of any vacuum pump of the vacuum pump unit VP, the air-driven valve 11c is controlled to be automatically closed, but the manual valve 11d is also provided to ensure safe operation even if malfunction of the air-driven valve 11c occurs. Instead of disposing the manual valve 11d, it is also possible to provide the air-driven valve 11c with a lock mechanism so that its valve position does not physically change even if there is a wrong air-driven operation.

Hydrogen gas sucked into the vacuum pump unit VP is exhausted through an exhaust pipe 11e, and merges with hydrogen gas exhausted from the adjacent module on the upstream side (i.e., the hydrogen pump module 11-3), and is then exhausted to the adjacent module on the downstream side (i.e., the hydrogen pump module 11-1). The exhaust pipe 11e is also provided with an air-driven valve 11c and a manual valve 11d.

The hydrogen pump module 11-2 is also provided with an air inlet port 11f, an exhaust port 11g, and a pump P. Therefore, when the pump P is operated, air is taken into the housing of the hydrogen pump module 11-2 from the outside of the hydrogen pump module 11-2 via the air inlet port 11f, and air is exhausted from the housing via the exhaust port 11g. Accordingly, even if hydrogen gas flows out from the vacuum pump unit VP, the intake pipe 11b, the exhaust pipe 11e, or the like, the gas is exhausted from the housing of the hydrogen pump module 11-2 together with air taken into the housing, so that the hydrogen gas is prevented from flowing out into the factory.

A hydrogen sensor H for detecting outflow of hydrogen gas from the vacuum pump unit VP, the intake pipe 11b, the exhaust pipe 11e, or the like is provided in the housing of the hydrogen pump module 11-2. When the hydrogen sensor H (i.e., a hydrogen detector) detects a hydrogen leak, a detection signal is sent to the control module 14. The control module 14 outputs a signal for notifying the user of the abnormal state. For example, the user may be notified of the hydrogen leak via a display unit or a speaker provided in at least one module of the exhaust system 10.

In addition, it is possible to perform control such that the supply of hydrogen to the EUV exposure apparatus 1 is cut off for safety on the basis of a detection signal output from the hydrogen sensor H or a notification signal output from the control module 14. The exhaust system 10 can also be safely stopped by closing, on the basis of the signal, the automatic valves (i.e., the air-driven valves) disposed upstream of all of the vacuum pumps for safety, and thus stopping the supply of hydrogen to the exhaust system 10.

The number of hydrogen pump modules included in the light-source-unit hydrogen pump module group 11 of the exhaust system 10 can be changed according to a user's request. For example, when the user's request has changed and a higher exhaust speed is required after the installation of the exhaust system 10, it is possible to add a hydrogen pump module(s) later. For example, three to eight hydrogen pump modules may be provided as the light-source-unit hydrogen pump modules.

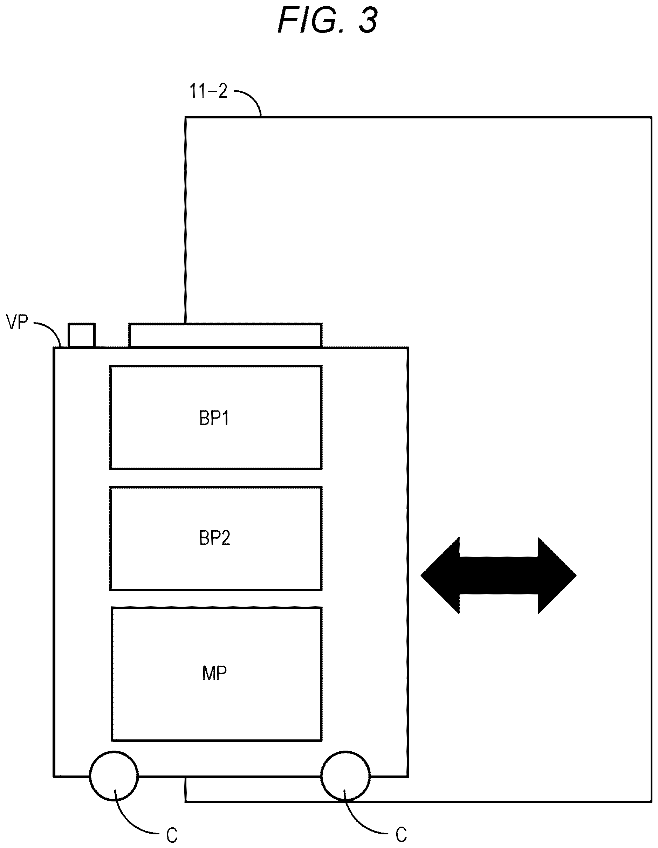

FIG. 3 is a diagram for illustrating a method of disposing the vacuum pump unit VP in the hydrogen pump module. As an example, the hydrogen pump module 11-2 included in the light-source-unit hydrogen pump module group 11 will be described, but the same applies to the other hydrogen pump modules included in the light-source-unit hydrogen pump module group 11 and the exposure-unit hydrogen pump module group 16.

As illustrated in FIG. 3, the vacuum pump unit VP of the present embodiment is provided with a caster (i.e., wheel) C. Conventionally, a plurality of vacuum pumps have not formed a single unit, but instead, two units have been formed, for example, which include a first unit having the first booster pump BP1, and a second unit having the second booster pump BP2 and the main pump MP. Therefore, it is required to use a jig for lifting up each unit to the height of an installation position within the housing, which poses a problem that it takes time to dispose the vacuum pumps.

Meanwhile, regarding the vacuum pump unit of the present embodiment, since the plurality of vacuum pumps (i.e., the first booster pump BP1, the second booster pump BP2, and the main pump MP) form a single unit (i.e., pump unit), and the vacuum pump unit VP is provided with the caster C, the vacuum pump unit VP can be easily disposed within the hydrogen pump module only by being moved in the horizontal direction. This can significantly reduce the time required for the work of disposing the vacuum pump unit VP.

Referring again to FIG. 1, hydrogen gas discharged from each of the vacuum pump units in the light-source-unit hydrogen pump module group 11 is transferred to the first oxygen sensor module 12 through a pump exhaust pipe.

### (First oxygen sensor module)

The first oxygen sensor module 12 is provided downstream of the light-source-unit hydrogen pump module group 11, and monitors the oxygen concentration of gas in the pump exhaust pipe. The gas in the pump exhaust pipe is basically composed of hydrogen gas, but there may be a case where a manifold or the pump exhaust pipe is damaged and air flows into the pump exhaust pipe from the outside. Therefore, the first oxygen sensor module 12 is configured to constantly measure the oxygen concentration of gas in the pump exhaust pipe, and generate an alarm signal according to the measured oxygen concentration. Specifically, the first oxygen sensor module 12 includes at least one oxygen sensor 12a attached to a branch line branching from the pump exhaust pipe, and an oxygen concentration monitoring device (not illustrated) that generates an alarm signal when the concentration of oxygen measured by the oxygen sensor 12a is higher than a threshold value. The measured value of the oxygen concentration is sent to the oxygen concentration monitoring device. The oxygen concentration monitoring device compares the measured oxygen concentration with a preset threshold value, and generates an alarm signal if the measured oxygen concentration is higher than the threshold value. The alarm signal is sent to the user's facility of the EUV exposure apparatus 1 and the exhaust system 10 so that the supply of hydrogen to the EUV exposure apparatus 1 is stopped. Specifically, a valve that supplies hydrogen to the EUV exposure apparatus 1 is closed so that the supply of hydrogen to the EUV exposure apparatus is stopped.

The gas in the pump exhaust pipe that has passed through the first oxygen sensor module 12 enters the first and second hydrogen treatment modules 18 and 19 via the utility module 13, the control module 14, the non-hydrogen pump module 15, the exposure-unit hydrogen pump module group 16, and the second oxygen sensor module 17.

### (Utility module)

The utility module 13 is a module for receiving cooling water and nitrogen gas (N₂) from a factory, and distributing the cooling water and the nitrogen gas to each module. The utility module 13 supplies the cooling water supplied from the factory side to each of the vacuum pump units in the light-source-unit hydrogen pump module group 11, the exposure-unit hydrogen pump module group 16, and the non-hydrogen pump module 15.

The cooling water is used to cool a casing, a motor, and the like of each vacuum pump unit VP, is then recovered from the utility module 13, so that it is discharged through a factory drainage line (not illustrated).

In addition, the utility module 13 supplies the nitrogen gas (N₂) supplied from the factory side to the light-source-unit hydrogen pump module group 11, the exposure-unit hydrogen pump module group 16, the non-hydrogen pump module 15, the first oxygen sensor module 12, and the second oxygen sensor module 17.

Nitrogen supplied from the utility module 13 (which has a single supply port) is branched to be supplied to the light-source-unit hydrogen pump module group 11, the exposure-unit hydrogen pump module group 16, the non-hydrogen pump module 15, the first and second oxygen sensor modules 12 and 17, and the valve-driving solenoid valves (i.e., the air-driven valves) of the respective modules. The nitrogen gas supplied to each vacuum pump unit VP is used for purging and shaft seal.

The nitrogen gas supplied from the utility module 13 to the non-hydrogen pump module 15 is used for shaft seal of the vacuum pump unit VP.

In addition, the nitrogen gas supplied from the utility module 13 to the first and second oxygen sensor modules 12 and 17 is used as a standard gas for accurately measuring the oxygen concentration.

The nitrogen gas used in the light-source-unit hydrogen pump module group 11, the exposure-unit hydrogen pump module group 16, the non-hydrogen pump module 15, and the first and second oxygen sensor modules 12 and 17 joins the pump exhaust pipe for hydrogen gas, and is then exhausted to the hydrogen treatment module 18 together with the hydrogen gas.

### (Control module)

The control module 14 supplies power supplied from a factory to each module, and also controls the pumps and sensors included in each module.

### (Non-hydrogen pump module)

The non-hydrogen pump module 15 is a module for exhausting air and nitrogen gas, other than hydrogen gas, discharged from a vacuum unit (i.e., a vacuum robot unit/a reticle stage unit) other than the light source unit 2 and the exposure unit 3 of the EUV exposure apparatus 1.

Specifically, the non-hydrogen pump module 15 includes a vacuum pump VP1 that sucks gas discharged from the vacuum robot unit, a vacuum pump VP2 that sucks gas discharged from the reticle stage unit, and a vacuum pump VP3 (not illustrated) as a spare pump to be used when the vacuum pump VP1 or the vacuum pump VP2 cannot be operated for some reason. When one of the vacuum pumps VP1 and VP2 fails to operate properly, the vacuum pump is switched to the vacuum pump VP3, which is a spare pump, so that a faulty pump can be replaced without stopping the operation of the vacuum robot unit and the reticle stage unit.

In the non-hydrogen pump module 15 of the present embodiment, the vacuum pump VP1 for the vacuum robot and the vacuum pump VP2 for the reticle stage share the spare pump (i.e., the vacuum pump VP3). Thus, the size of the non-hydrogen pump module 15 can be reduced as compared with a case where a single spare pump is provided for each of the vacuum pump VP1 and the vacuum pump VP2.

Gas discharged from the vacuum pumps VP1 to VP3 of the non-hydrogen pump module 15 is exhausted to the outside of the factory (i.e., in the atmosphere) through a factory exhaust pipe (not illustrated).

### (Exposure-unit hydrogen pump modules)

Hydrogen gas discharged from the exposure unit 3 of the EUV exposure apparatus 1 is sucked into the exposure-unit hydrogen pump module group 16 through a manifold. The basic configuration of the hydrogen pump modules 16-1 to 16-n included in the exposure-unit hydrogen pump module group 16 is similar to that of the hydrogen pump modules 11-1 to 11-n included in the light-source-unit hydrogen pump module group 11.

Each of the hydrogen pump modules included in the exposure-unit hydrogen pump module group 16 includes a vacuum pump unit VP that sucks hydrogen gas discharged from the exposure unit 3 of the EUV exposure apparatus 1. A vacuum pump mounted in the vacuum pump unit VP is a positive-displacement dry vacuum pump, for example, but its specific configuration is not limited to a particular configuration. The exhaust system 10 of the present embodiment is configured such that hydrogen gas discharged from the exposure unit 3 of the EUV exposure apparatus 1 is sucked into the exposure-unit hydrogen pump module group 16. The exposure-unit hydrogen pump module group 16 may include a plurality of hydrogen pump modules (it includes n hydrogen pump modules 16-1 to 16-n in the example illustrated in FIG. 1). Accordingly, even when the operation of one hydrogen pump module is stopped for maintenance, for example, the suction of hydrogen gas can be continued with the other hydrogen pump modules. Therefore, maintenance and the like of the hydrogen pump modules can be performed without stopping the operation of the EUV exposure apparatus 1.

Hydrogen gas discharged from the vacuum pump units VP in the exposure-unit hydrogen pump module group 16 is transferred to the second oxygen sensor module 17 through a pump exhaust pipe.

### (Second oxygen sensor module)

The second oxygen sensor module 17 is provided downstream of the exposure-unit hydrogen pump module group 16, and monitors the oxygen concentration of gas in the pump exhaust pipe. The gas in the pump exhaust pipe is basically composed of hydrogen gas, but there may be a case where a manifold or the pump exhaust pipe is damaged and air flows into the pump exhaust pipe from the outside. Therefore, the second oxygen sensor module 17 is configured to constantly measure the oxygen concentration of gas in the pump exhaust pipe, and generate an alarm signal according to the measured oxygen concentration. Specifically, the second oxygen sensor module 17 includes at least one oxygen sensor 17a attached to a branch line branching from the pump exhaust pipe, and an oxygen concentration monitoring device (not illustrated) that generates an alarm signal when the concentration of oxygen measured by the oxygen sensor 17a is higher than a threshold value. The measured value of the oxygen concentration is sent to the oxygen concentration monitoring device. The oxygen concentration monitoring device compares the measured oxygen concentration with a preset threshold value, and generates an alarm signal if the measured oxygen concentration is higher than the threshold value. The alarm signal is sent to the EUV exposure apparatus 1 on the upstream side so that the supply of hydrogen to the EUV exposure apparatus 1 is stopped.

The gas in the pump exhaust pipe that has passed through the second oxygen sensor module is transferred to the first and second hydrogen treatment modules.

### (Hydrogen treatment module)

The hydrogen treatment modules (i.e., the first and second hydrogen treatment modules 18 and 19) are apparatuses for treating hydrogen gas discharged from the light source unit 2 side (i.e., the light-source-unit hydrogen pump module group 11) and the exposure unit 3 side (i.e., the exposure-unit hydrogen pump module group 16) of the EUV exposure apparatus 1. Specifically, each of the first and second hydrogen treatment modules 18 and 19 includes a hydrogen dilution apparatus that dilutes hydrogen gas to a concentration equal to or lower than the explosion lower limit, or a combustion-type exhaust gas treatment apparatus (i.e., a combustion abatement apparatus) that performs combustion treatment on hydrogen gas by bringing it into contact with a flame. When the first hydrogen treatment module 18 and/or the second hydrogen treatment module 19 are/is used as a hydrogen dilution apparatus, air exhausted from the housing of each module is used as dilution air. Accordingly, the user of the exhaust system 10 does not need to separately prepare a facility for supplying dilution air to the hydrogen dilution apparatus.

Regarding the first and second hydrogen treatment modules 18 and 19, when the first hydrogen treatment module 18 mainly treats hydrogen gas and the first hydrogen treatment module 18 is unable to operate for some reason, the second hydrogen treatment module 19 treats hydrogen gas instead of the first hydrogen treatment module 18 as backup. When the second hydrogen treatment module 19 is unable to operate for some reason, either, the hydrogen gas is directly exhausted to the outside of the factory (i.e., in the atmosphere) through a factory exhaust pipe (not illustrated). Note that both the first and second hydrogen treatment modules 18 and 19 may be configured to constantly treat hydrogen gas.

The gas treated by the first hydrogen treatment module 18 and/or the second hydrogen treatment module 19 is exhausted to the outside of the factory (i.e., in the atmosphere) through a factory exhaust pipe (not illustrated).

As described above, the exhaust system 10 according to the present embodiment includes the modules (i.e., the light-source-unit hydrogen pump module group 11, the first oxygen sensor module 12, the utility module 13, the control module 14, the non-hydrogen pump module 15, the exposure-unit hydrogen pump module group 16, the second oxygen sensor module 17, the first hydrogen treatment module 18, and the second hydrogen treatment module 19) that are each separable. Thus, it is possible to increase or decrease the number of the modules according to a request of the user of the exhaust system 10.

### <Second embodiment>

Next, an exhaust system 10A according to a second embodiment will be described. The exhaust system 10A according to the second embodiment differs from the exhaust system 10 according to the first embodiment in that the exhaust system 10A includes a hydrogen recovery module 20 for recovering hydrogen gas discharged from the EUV exposure apparatus 1 for reuse in the EUV exposure apparatus 1, and also includes a hydrogen treatment module 19 as backup, whereas the exhaust system 10 is configured such that hydrogen gas discharged from the EUV exposure apparatus is treated by the first and second hydrogen treatment modules.

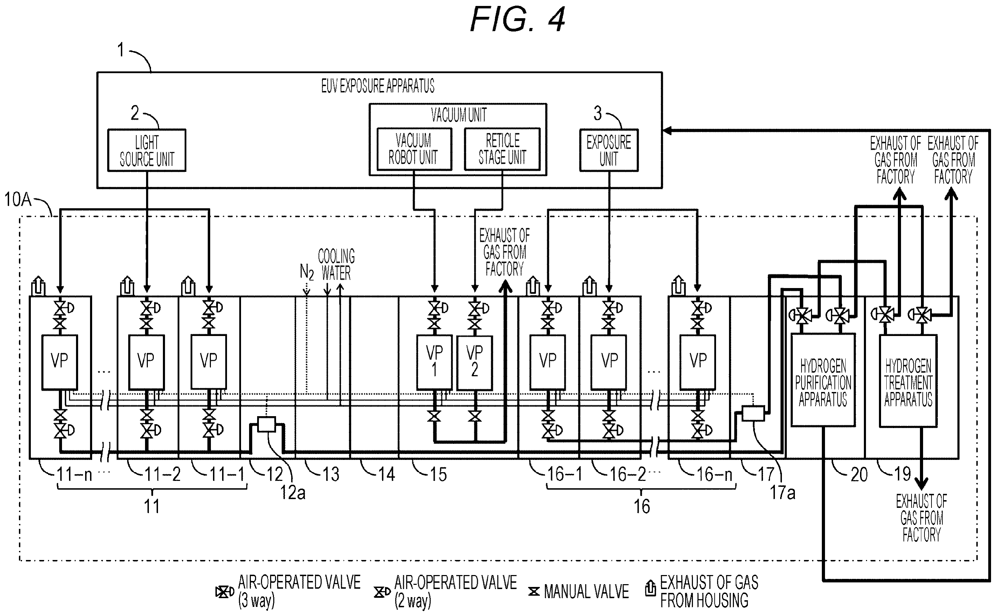

FIG. 4 is a diagram illustrating an example of a schematic configuration of the exhaust system 10A according to the second embodiment. The same components as those of the first embodiment are denoted by the same reference numerals, and the description thereof will be omitted.

As illustrated in FIG. 4, hydrogen gas discharged from the light source unit 2 side (i.e., the light-source-unit hydrogen pump module group 11) and the exposure unit 3 side (i.e., the exposure-unit hydrogen pump module group 16) of the EUV exposure apparatus 1 is first transferred to the hydrogen recovery module 20.

The hydrogen recovery module 20 has a hydrogen purification apparatus for purifying hydrogen gas, and hydrogen gas purified by the hydrogen purification apparatus is transferred to the EUV exposure apparatus 1 through a hydrogen transfer tube for reuse.

When the hydrogen purification apparatus is unable to operate for some reason, the hydrogen gas is transferred to the hydrogen treatment module 19, and thus is treated by the hydrogen treatment apparatus (i.e., the hydrogen dilution apparatus or the combustion-type exhaust gas treatment apparatus). When the hydrogen treatment apparatus is unable to operate for some reason, either, the hydrogen gas is directly exhausted to the outside of the factory (in the atmosphere) through a factory exhaust pipe (not illustrated).

As described above, in the exhaust system of the present embodiment, hydrogen gas discharged from the EUV exposure apparatus 1 can be recovered for reuse. Thus, the hydrogen gas can be effectively used without being discharged to the atmosphere as exhaust gas.

### <Third embodiment>

Next, an exhaust system 10B according to a third embodiment will be described. The exhaust system 10B according to the third embodiment differs from the exhaust system 10 according to the first embodiment in that the exhaust system 10B includes a fuel cell module 21 for generating power using hydrogen gas discharged from the EUV exposure apparatus 1, and also includes a hydrogen treatment module 19 as backup, whereas the exhaust system 10 is configured such that hydrogen gas discharged from the EUV exposure apparatus 1 is treated by the first and second hydrogen treatment modules 18 and 19.

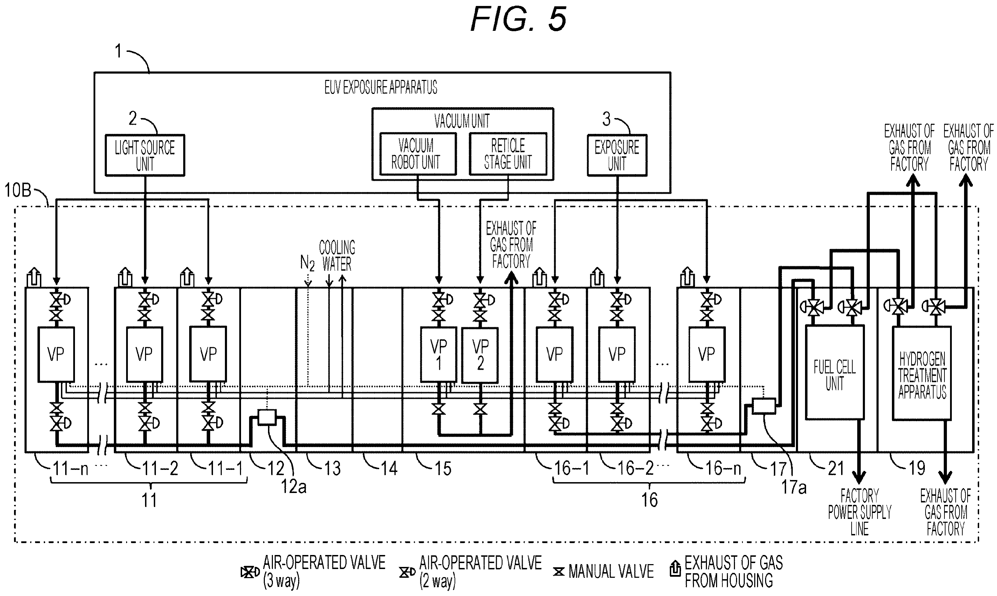

FIG. 5 is a diagram illustrating an example of a schematic configuration of the exhaust system 10B according to the third embodiment. The same components as those of the first embodiment are denoted by the same reference numerals, and the description thereof will be omitted.

As illustrated in FIG. 5, hydrogen gas discharged from the light source unit 2 side (i.e., the light-source-unit hydrogen pump module group 11) and the exposure unit 3 side (i.e., the exposure-unit hydrogen pump module group 16) of the EUV exposure apparatus 1 is first transferred to the fuel cell module 21.

The fuel cell module 21 includes a fuel cell unit for generating power using hydrogen gas. The fuel cell unit is supplied with hydrogen gas discharged from the light source unit 2 side (i.e., the light-source-unit hydrogen pump module group 11) and the exposure unit 3 side (i.e., the exposure-unit hydrogen pump module group 16) of the EUV exposure apparatus 1. The fuel cell unit is also supplied with oxygen in the air through an intake port (not illustrated), and power is generated using the hydrogen gas and the oxygen. Power generated by the fuel cells is supplied to a power supply facility of a factory (i.e., a factory power supply line) through an electric cable.

When the fuel cell unit cannot be used for some reason, the hydrogen gas is transferred to the hydrogen treatment module 19, and thus is treated by the hydrogen treatment apparatus (i.e., the hydrogen dilution apparatus or the combustion-type exhaust gas treatment apparatus). When the hydrogen treatment apparatus is unable to operate for some reason, either, the hydrogen gas is directly exhausted to the outside of the factory (in the atmosphere) through a factory exhaust pipe (not illustrated).

As described above, in the exhaust system 10B of the present embodiment, the fuel cell module 21 is configured to generate power using hydrogen gas discharged from the EUV exposure apparatus 1. Thus, the hydrogen gas can be effectively used without being discharged to the atmosphere as exhaust gas.

The present disclosure is not limited to the above-described embodiments, and can be embodied by modifying the components without departing from the gist of the present disclosure in the implementation stage. In addition, various disclosures can be formed by appropriately combining some of the plurality of components disclosed in the above-described embodiments. For example, some components may be deleted from all of the components illustrated in the embodiments. Furthermore, some of the components in different embodiments may be appropriately combined.

### Other Embodiments

Embodiment(s) of the present disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and the like.

While the present disclosure has been described with reference to exemplary embodiments, it is to be understood that the disclosure is not limited to the disclosed exemplary embodiments. The scope of the following claims is to be accorded the broadest interpretation so as to encompass all such modifications and equivalent structures and functions.

This application claims the benefit of Japanese Patent Applications No. 2025-31336, filed February 28, 2025, which is hereby incorporated by reference herein in its entirety.

## Claims

1. An exhaust system for exhausting hydrogen discharged from an EUV exposure apparatus, the exhaust system comprising:
a light-source-unit hydrogen pump module having a vacuum pump unit, the vacuum pump unit being configured to suck hydrogen discharged from a light source unit of the EUV exposure apparatus;
a first oxygen sensor module configured to measure an oxygen concentration of gas discharged from the light-source-unit hydrogen pump module;
an exposure-unit hydrogen pump module having a vacuum pump unit, the vacuum pump unit being configured to suck hydrogen discharged from an exposure unit of the EUV exposure apparatus;
a second oxygen sensor module configured to measure an oxygen concentration of gas discharged from the exposure-unit hydrogen pump module;
a hydrogen treatment module configured to treat hydrogen gas discharged from the first and second oxygen sensor modules;
a utility module configured to supply cooling water and/or nitrogen gas to each module; and
a control module configured to supply power to each module, and control each module.

2. The exhaust system according to claim 1, wherein
the hydrogen treatment module includes a hydrogen dilution apparatus configured to dilute the hydrogen gas, and
air discharged from a housing of each module is supplied to the hydrogen dilution apparatus as dilution air.

3. The exhaust system according to claim 1, wherein
the hydrogen treatment module includes a combustion-type exhaust gas treatment apparatus configured to perform combustion treatment on the hydrogen gas.

4. The exhaust system according to claim 1, further comprising
a hydrogen recovery module configured to recover the hydrogen gas for reuse in the EUV exposure apparatus, wherein
the hydrogen recovery module includes a hydrogen purification apparatus configured to purify the recovered hydrogen gas.

5. The exhaust system according to claim 1, further comprising
a fuel cell module configured to generate power using the hydrogen gas.

6. The exhaust system according to claim 1, wherein
the vacuum pump unit has a main pump, a first booster pump, and a second booster pump, and has a caster at a bottom.

7. The exhaust system according to claim 1, further comprising
a non-hydrogen pump module configured to discharge non-hydrogen gas discharged from a device other than the light source unit and the exposure unit of the EUV exposure apparatus.
